# EUROPEAN PATENT SPECIFICATION

(11) **EP 1 644 490 B1**
(45) Date of publication and mention of the grant of the patent: **08.06.2016**
(21) Application number: 04743398.2
(22) Date of filing: 13.07.2004
(51) Int. Cl.: C12N 5/10

(54) **TRANSGENIC CELLS**
TRANSGENE ZELLEN
CELLULES TRANSGENIQUES

(30) Priority: 16.07.2003 GB 0316629
(43) Date of publication of application: 12.04.2006
(73) Proprietor: THE UNIVERSITY OF YORK, Heslington, York YO10 5DD (GB)
(72) Inventor: GRAHAM, Ian, Heslington, York YO1 5DD (GB); TONON, Thierry, 29600 Morlaix (FR)
(74) Representative: Herzog, Fiesser & Partner Patentanwälte PartG mbB
(86) International application number: PCT/GB2004/003057
(87) International publication number: WO 2005/007845

(56) References cited:
- WO-A-02/08401
- WO-A-02/090493
- WO-A-03/078639
- TONON T ET AL: "Long chain polyunsaturated fatty acid production and partitioning to triacylglycerols in four microalgae" PHYTOCHEMISTRY, PERGAMON PRESS, GB, vol. 61, no. 1, September 2002 (2002-09), pages 15-24, XP004374192 ISSN: 0031-9422
- DATABASE EMBL [Online] 23 September 1998 (1998-09-23), "GH11554.5prime GH Drosophila melanogaster head pOT2 Drosophila melanogaster cDNA clone GH11554 5 similar to Baldspot: FBan0003971 GO:[plasma membrane (GO:0005886)] located on: 3L 73B1-73B1;: 08/12/2002, mRNA sequence." XP002312674 retrieved from EBI accession no. EM_PRO:AI134173 Database accession no. AI134173
- ABBADI A ET AL: "Transgenic oilseeds as sustainable source of nutritionally relevant C20 and C22 polyunsaturated fatty acids?" EUROPEAN JOURNAL OF LIPID SCIENCE AND TECHNOLOGY, WILEY VCH VERLAG, WEINHEIM, DE, vol. 103, no. 2, February 2001 (2001-02), pages 106-113, XP002228744 ISSN: 1438-7697
- JGI: "Thalassiosira pseudonana; Advanced search, Search terms : elongase; ELO1"[Online] XP002312673 Retrieved from the Internet: URL:http://genome.jgi-psf.org:8080/annotat or/servlet/jgi.annotation.Annotation?pStat eVar=Search&pProteinId=0&pStart=0&pEnd=&pR andom=88548&pDbList=%2Cthaps1&pASearch=elo ngase&pSearchType=model&pSig=evalue&pNumer ic=1.0e-20&pDb=thaps1&pPerPage=25&pSearchR adio=reg> [retrieved on 2005-01-10]
- JGI: "Thalassiosira pseudonana; Advanced search, Search terms : elongase; ELO2"[Online] XP002314938 Retrieved from the Internet: URL:http://genome.jgi-psf.org:8080/annotat or/servlet/jgi.annotation.Annotation?pStat eVar=Search&pProteinId=0&pStart=0&pEnd=&pR andom=88548&pDbList=%2Cthaps1&pASearch=elo ngase&pSearchType=model&pSig=evalue&pNumer ic=1.0e-20&pDb=thaps1&pPerPage=25&pSearchR adio=reg> [retrieved on 2005-01-10]
- JGI: "Thalassiosira pseudonana; Advanced search, Search terms : elongase; ELO3"[Online] XP002314939 Retrieved from the Internet: URL:http://genome.jgi-psf.org:8080/annotat or/servlet/jgi.annotation.Annotation?pStat eVar=Search&pProteinId=0&pStart=0&pEnd=&pR andom=88548&pDbList=%2Cthaps1&pASearch=elo ngase&pSearchType=model&pSig=evalue&pNumer ic=1.0e-20&pDb=thaps1&pPerPage=25&pSearchR adio=reg> [retrieved on 2005-01-10]

## Description

The invention relates to transgenic cells comprising nucleic acid molecules that comprise nucleic acid sequences which encode enzymes involved in the biosynthesis of n-3 fatty acids.

DHA, an example of a n-3 fatty acid can be obtained directly from the diet or derived from metabolism of dietary linoleic and α-linolenic acid. To obtain sufficient amounts of this fatty acid humans have to eat foods rich in DHA. Currently the principle dietary source of DHA is fish or fish oil. However, this has many inherent problems; fish accumulate pollutants, the extracted oil has an unpleasant odour, there is a difficulty in controlling the proportion of specific desirable fatty acids from this source and since fish are a declining resource the market demand for DHA is not being met. Also, vegetarians do not have an obvious alternative food source to fish and therefore either do without DHA or have to take pure supplements.

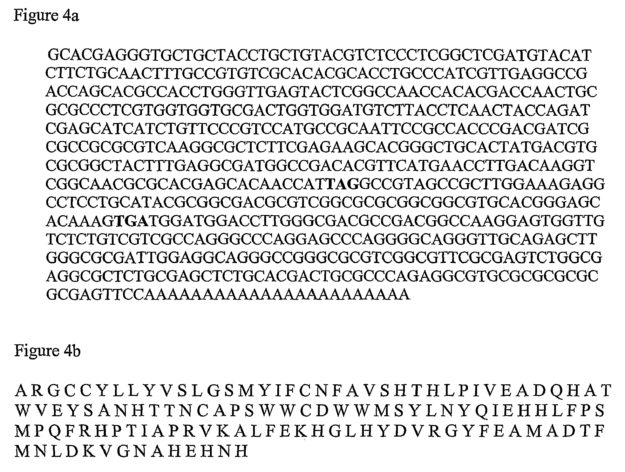

Long chain polyunsaturated fatty acids (LPUFAs) are derived from the essential fatty acids (EFA) linoleic acid (18:2n-6) and α-linolenic acid (18:3n-3), the parent compounds of the so-called omega-3 and omega-6 EFA families by an alternating series of desaturation and elongation reactions (Haag, 2001), see Figure 4. The major metabolite product of the n-6 pathway in mammals is arachidonic acid (AA) (20:4n-6), whilst the major end products of the n-3 pathway are eicosapentaenoic acid (EPA) (20:5n-3) and docosahexaenoic acid (DHA, 22:6n-3). The biosynthesis of 18:3n-3 from 18:4n-3 involves the action of a Δ6 desaturase (Horrobin DF, 1992). This is followed by an elongation reaction to 20:4n-3 (Sprecher *et al.,* 1995) and a Δ5 desaturation to 20:5n-3 (Sprecher *et al.,* 1995). The conventional view is that there is then a further elongation step converting 20:5n-3 to 22:5n-3, which is then followed by a final desaturation step involving the activity of a Δ4 desaturase to produce DHA (22:6n-3).

During evolution humans have consumed a diet containing approximately equal ratio of n-3 and n-6 essential fatty acids (1-2:1), but the last 100-150 years has seen a growing trend in Western diets towards the consumption of more n-6 fatty acids, resulting in an alteration of the ratio to 30:1 (Simonpolous, 1999). Whilst an increased intake of n-6 fatty acids is characterised by cardiovascular problems such as increased blood viscosity, vasospasm and vasoconstriction, the n-3 fatty acids are associated with health promoting properties. For example n-3 fatty acids have been described as anti-inflammatory, antithrombotic, antiarrhythmic, hypolipidemic and vasodilatory (Simonpolous, 1999). As such the role of DHA in the prevention and/or treatment of diseases such as coronary heart disease, hypertension, type II diabetes, ocular diseases, arthritis, cystic fibrosis and schizophrenia and has been the focus of a great deal of medical research.

The effect of n-3 polyunsaturated fatty acids in the cardiovascular diseases has shown that dietary intake of DHA can lower the risk of myocardial infarction, hypertension and complications associated with cardiac surgery. A number of population studies have correlated the dietary intake of DHA with cardiovascular risk factors. For instance, a study of a population of Inuits in Canada (426 subjects aged 18-74 yr), who traditionally consume large amounts of marine foods rich in n-3 fatty acids, showed that n-3 fatty acids, such as DHA were positively associated with HDL-cholesterol concentrations and inversely associated with triacylglycerol concentrations and the ratio of total to HDL cholesterol (Dewailly *et al.,* 2001). It was concluded that the high dietary intake of n-3 fatty acids in the Inuit diet was probably responsible for the low mortality rate from ischemic heart disease in this population.

Essential fatty acids are structural components of all tissues and are indispensable for cell membrane synthesis. The brain, retina and other neural tissues have been found to be particularly rich in DHA, where it is involved in neural development and maturation of sensory systems (Uauy *et al.,* 2000). A large body of research comparing infants fed with breast milk compared to formula milk, which is deficient in DHA and other omega 3- fatty acids, has concluded that the presence of DHA is critical during the development of the newborn (Horrocks *et a*/.,1999). DHA forms 25% of the fatty acid complement of the glycosphingolipids of the brain and is an important component of the rods of the retina, and therefore a deficiency in DHA during infant development has been associated with a reduction in cognitive function and visual acuity. Furthermore, deficiencies in DHA have been associated with foetal alcohol syndrome, attention deficit hyperactivity disorder, cystic fibrosis, phenylketonuria and adrenoleukodystrophy.

To meet this increased demand for n-3 fatty acids such as DHA a number of approaches have been attempted. Methods to enhance the DHA content of meat by manipulating animal feed have been met with little success. The cultivation of marine micro-organisms such as the *Crypthecodinium cohnii* and *Schizochytrium* sp, which are rich sources of DHA has also met with some limited success as the cultivation of algae is technically demanding and costly (Ashford *et al.,* 2000).

There has been limited research focused on the identification of genes involved in the biosynthesis of n-3 fatty acids in algae. In one report the identification of a cDNA encoding a novel C18- Δ⁹ polyunsaturated fatty acid-specific elongating activity from the docosahexaenoic acid (DHA)-producing microalga, *Isochrysis galbana* was described (Qi *et al.,* 2002). This 30 kDa elongase, designated IgASE1, shares only limited homology to animal and fungal proteins with elongating activity. When IgASE1 was expressed in the yeast *Saccharomyces cerevisiae,* it was shown to specifically elongate the C18- Δ⁹ polyunsaturated fatty acids, linoleic acid (C18:2n-6, Δ^{9,12} and alpha-linolenic acid (C18:3, Δ^{9,12,15}), to eicosadienoic acid (C20:2, Δ^{11,14}) and eicosatrienoic acid (C20:3 Δ^{11,14,17}), respectively. It was concluded that a major route for eicosapentaenoic acid (C20:5 Δ^{5,8,11,14,17}), and docosahexaenoic acid (C22:6 Δ^{4,7,10,13,16,19}) syntheses in *I. galbana* may involve a Δ⁸ desaturation pathway.

Δ6 and Δ5 desaturases are microsomal enzymes that are thought to be a component of a three-enzyme system that includes NADH*-cytochrome b₅* reductase, cytochrome *b₅,* and the respective desaturase (Sprecher, 1981).

A number of Δ6 and Δ5 desaturases have been identified. In plants such as the herb, borage *(Borago officinalis),* the Δ6 desaturase has been identified (Sayanova *et al.,* 1997). Δ6 and Δ5 desaturases have been identified in humans (Hyekyung *et al.,* 1999 and Cho *et al.,* 1999, respectively), in animals such as the nematode; *Caenorhabditis elegans* (Michaelson *et al.,* 1998 and Napier *et al.,* 1998) and in eukaryotic microorganisms such as the fungus *Mortierella alpina* (Huang *et al.,* 1999 and Knutzon *et al.,* 1998). In the human, Δ6 and Δ5 desaturase activities have been found in skeletal muscle, lung, placenta, kidney and pancreas, but are expressed at the highest levels in the liver, brain and heart (Hyekyung *et al.,* 1999). In all these tissues however, Δ6 desaturase activity was found to be higher than that of Δ5 desaturase. The genes for both of the enzymes reside on chromosome 11, in a reverse orientation, being separated by <11,000 base pairs *(Hyekyung et al.,* 1999). A Δ4 desaturase that can introduce a double bond at position 4 of 22:5 n-3 and 22:4 n-6 resulting in the production of DHA and docosapentanoic acid has been identified in the marine fungi *Thraustochytrium* sp (Qiu *et al.,* 2001).

Cellular storage of fatty acids in triacylglycerol requires that the fatty acids are first activated to their acyl-CoA esters through the action of acyl-CoA synthetase enzyme. Acyl-CoA's are produced by acyl-CoA synthetase from fatty acid, ATP and Coenzyme A. Acyl-CoA synthetases can exhibit substrate specificity for different chain length or different degrees of saturation of the fatty acid. For example an arachidonate (20:4 n-6)-preferring acyl-CoA synthetase has been identified in rat (Kang *et al.,* 1997). This enzyme has a high affinity for arachidonate and EPA and low affinity for palmitate. Several isoforms of acyl-CoA synthetases have also been identified in *Arabidopsis* (Schnurr *et al.,* 2000).

Acyl CoA:diacyglycerol acyltransferase (DGAT) catalyses the final enzymatic step in the production of triacylglycerols in plants, fungi and mammals . The ezyme is responsible for transferring an acyl group from acyl-CoA to the *sn*-3 position of 1,2-diacylglycerol (DAG) to form triacylglycerol (TAG). The first cloning of a DGAT gene was from mouse (Cases *et al.,* 1998). An *Arabidopsis* homologue of the mouse DGAT gene was subsequently reported and found to be present as a single copy gene (Hobbs *et al.,* 1999). Jako *et al.,* (2001) showed that the *Arabidopsis Tag1* mutant which is disrupted in the DGAT gene and has a fatty acid and reduced oil phenotype can be complemented by expression of the DGAT cDNA. Jako *et al.,* (2001) also showed that seed-specific over-expression of the DGAT cDNA in wild-type *Arabidopsis* enhances oil deposition and average seed weight thus confirming the important role of DGAT in regulating the quantity of seed triacylglycerols and the sink size in developing seeds. Protein purification based studies on the oleaginous fungus *Mortierella ramanniana* resulted in the identification of a second class of proteins involved in TAG production that are encoded by the *DGAT2* gene family that are unrelated to the previously identified *DGAT1* gene family (Lardizabal *et al.,* 2001). A human homologue of the *Mortierella ramanniana DGAT2* gene has been also been identified (Cases *et al.,* 2001). Substrate specifities of the different families have yet to be determined.

Whilst higher plants do not typically biosynthesise LPUFAs such as DHA, they are an attractive target for genetic manipulation, particularly the low cost production of DHA in the vegetable oil of a crop such as oilseed rape. There have been no reports of higher plants that biosynthesise DHA, a number of attempts to introduce algal genes in order to manipulate the biosynthetic capacity of oil seed plants that produce LPUFAs have been reported. These have included the introduction of desaturases into transgenic plants to increase the production of DHA, EPA and also stearidonic acid (18:4n-3).

We herein disclose nucleic acid molecules which encode enzymes involved in n-3 fatty acid metabolism and the manipulation of these sequences and the biochemical pathways which comprise enzymes encoded by these sequences, to provide an alternative dietary source of n-3 fatty acids and, in particular, DHA. The sequences encode n-3 fatty acid elongase,desaturase, acyl CoA synthetase and diacylglycerol acyltransferase activities.

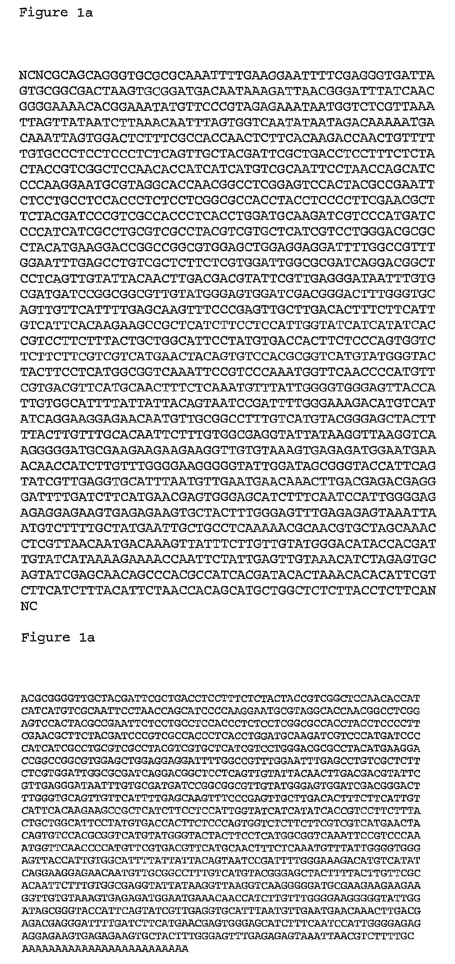

According to an aspect of the invention there is provided a transgenic plant cell comprising a nucleic acid molecule selected from the group consisting of:
(i) a DNA molecule selected from the DNA sequences as represented in Figure 1a;
(ii) a DNA molecule which anneals under stringent conditions to the sequences identified in (i) above and which encode a polypeptide which has fatty acid elongase activity.
(iii)

In a preferred embodiment of the invention said nucleic acid molecules are isolated from an algal species.

Preferably said algal species is selected from the group consisting of: *Amphidinium carterae, Amphiphora hyalina, Amphiphora sp., Chaetoceros gr acilis, Coscinodiscus sp., Crypthecodinium cohnii, Cryptomonas sp., Cylindrotheca fusiformis, Haslea ostrearia, Isochrysis galbana, Nannochlor opsis oculata, Navicula sp., Nitzschia closterium, Pavlova lutheri, Phaeodactylum tricornutum, Prorocentrum minimum, Rhizosolenia setigera, Skeletonema costatum, Skeletonema sp., Tetraselmis tetrathele, Thalassiosira nitzschioides, Thalassiosira heterophorma, Thalassiosira pseudonana, Thalassiosira stellaris.*

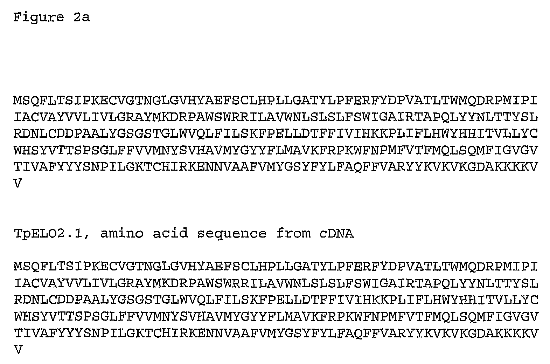

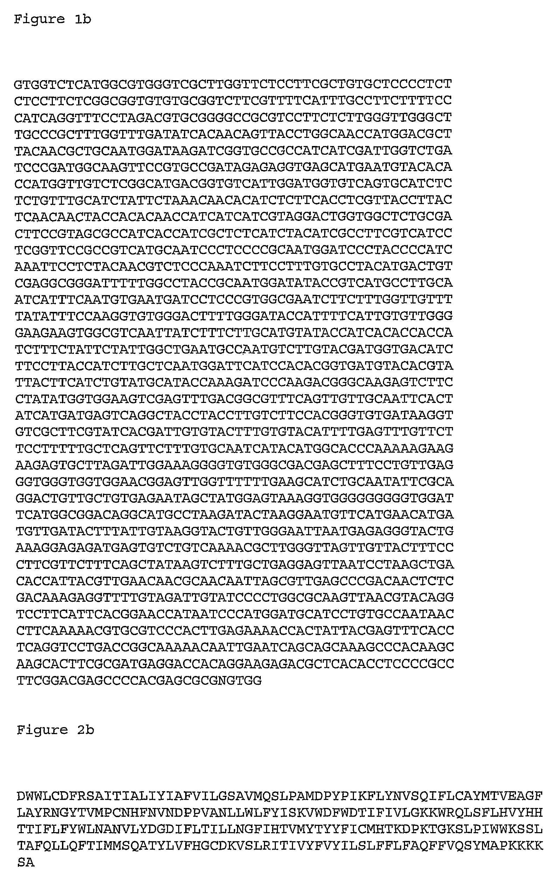

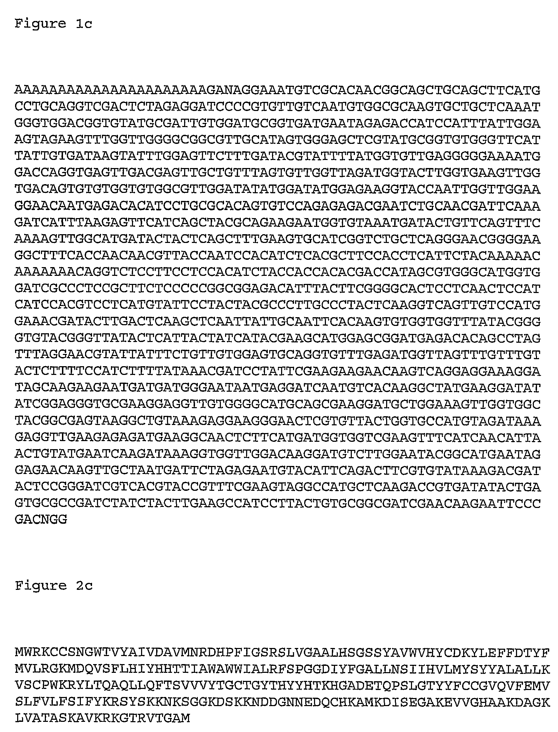

In a further preferred embodiment of the invention said polypeptide is a variant polypeptide having fatty elongase activity and comprises the amino acid sequence represented in Figure 2a which sequence has been modified by deletion, addition or substitution of at least one amino acid residue and has at least 75% sequence identity with the amino acid sequence in Figure 2a wherein said modification enhances the enzyme activity of said polypeptide.

A variant polypeptide may differ in amino acid sequence by one or more substitutions, additions, deletions, truncations that may be present in any combination. Among preferred variants are those that vary from a reference polypeptide by conservative amino acid substitutions. Such substitutions are those that substitute a given amino acid by another amino acid of like characteristics. The following non-limiting list of amino acids are considered conservative replacements (similar): a) alanine, serine, and threonine; b) glutamic acid and aspartic acid; c) asparagine and glutamine d) arginine and lysine; e) isoleucine, leucine, methionine and valine and f) phenylalanine, tyrosine and tryptophan. Most highly preferred are variants that retain or enhance the same biological function and activity as the reference polypeptide from which it varies.

A functionally equivalent polypeptide(s) according to the invention is a variant wherein one in which one or more amino acid residues are substituted with conserved or non-conserved amino acid residues, or one in which one or more amino acid residues includes a substituent group. Conservative substitutions are the replacements, one for another, among the aliphatic amino acids Ala, Val, Leu and He; interchange of the hydroxyl residues Ser and Thr; exchange of the acidic residues Asp and Glu; substitution between amide residues Asn and Gln; exchange of the basic residues Lys and Arg; and replacements among aromatic residues Phe and Tyr.

In addition, the invention features polypeptide sequences having at least 75% identity with the polypeptide sequences as herein disclosed. In one embodiment, the polypeptides have at least 85% identity, more preferably at least 90% identity, even more preferably at least 95% identity, still more preferably at least 97% identity, and most preferably at least 99% identity with the amino acid sequences illustrated herein.

Ideally said modified polypeptide has enhanced fatty acid elongase activity

In a further preferred embodiment of the invention said polypeptide comprises the amino acid sequence represented in Figure 2a. Preferably said polypeptide consists of the amino acid sequence represented in Figure 2a.

A vector including at least one nucleic acid molecule wherein said nucleic acid molecule is selected from the group consisting of:
i) a DNA molecule consisting of a DNA sequence as represented in Figures 1a, 1b or 1c;
ii) a DNA molecule which hybridises to the sequences identified in (i) above and which encode a polypeptide which has fatty acid elongase activity; and
iii) DNA molecules consisting of DNA sequences that are degenerate as a result of the genetic code to the DNA sequence defined in (i) and (ii) is disclosed herein.

A vector including nucleic acid (s) according to the invention need not include a promoter or other regulatory sequence, particularly if the vector is to be used to introduce the nucleic acid into cells for recombination into the genome for stable transfection.

Preferably the nucleic acid in the vector is operably linked to an appropriate promoter or other regulatory elements for transcription in a host cell such as a prokaryotic, (e.g. bacterial), or eukaryotic (e.g. fungal, plant, mammalian or insect cell). The vector may be a bi-functional expression vector which functions in multiple hosts. In the example of nucleic acids encoding polypeptides according to the invention this may contain its native promoter or other regulatory elements and in the case of cDNA this may be under the control of an appropriate promoter or other regulatory elements for expression in the host cell.

By "promoter" is meant a nucleotide sequence upstream from the transcriptional initiation site and which contains all the regulatory regions required for transcription. Suitable promoters include constitutive, tissue-specific, inducible, developmental or other promoters for expression in plant cells comprised in plants depending on design. Such promoters include viral, fungal, bacterial, animal and plant-derived promoters capable of functioning in plant cells.

Constitutive promoters include, for example CaMV 35S promoter (Odell et al (1985) Nature 313, 9810-812); rice actin (McElroy et al (1990) Plant Cell 2: 163-171); ubiquitin (Christian et al . (1989) Plant Mol. Biol. 18 (675-689); pEMU (Last et al (1991) Theor Appl. Genet. 81: 581-588); MAS (Velten et al (1984) EMBO J. 3. 2723-2730); ALS promoter (U.S. Application Seriel No. 08/409,297), and the like. Other constitutive promoters include those in U.S. Patent Nos. 5,608,149; 5,608,144; 5,604,121; 5,569,597; 5,466,785; 5,399,680, 5,268,463; and 5,608,142.

Chemical-regulated promoters can be used to modulate the expression of a gene in a plant through the application of an exogenous chemical regulator. Depending upon the objective, the promoter may be a chemical-inducible promoter, where application of the chemical induced gene expression, or a chemical-repressible promoter, where application of the chemical represses gene expression. Chemical-inducible promoters are known in the art and include, but are not limited to, the maize In2-2 promoter, which is activated by benzenesulfonamide herbicide safeners, the maize GST promoter, which is activated by hydrophobic electrophilic compounds that are used as pre-emergent herbicides, and the tobacco PR-1a promoter, which is activated by salicylic acid. Other chemical-regulated promoters of interest include steroid-responsive promoters (see, for example, the glucocorticoid-inducible promoter in Schena et al (1991) Proc. Natl. Acad. Sci. USA 88: 10421-10425 and McNellie et al. (1998) Plant J. 14(2): 247-257) and tetracycline-inducible and tetracycline-repressible promoters (see, for example, Gatz et al. (1991) Mol. Gen. Genet. 227: 229-237, and US Patent Nos. 5,814,618 and 5,789,156, herein incorporated by reference.

Where enhanced expression in particular tissues is desired, tissue-specific promoters can be utilised. Tissue-specific promoters include those described by Yamamoto et al. (1997) Plant J. 12(2): 255-265; Kawamata et al (1997) Plant Cell Physiol. 38(7): 792-803; Hansen et al (1997) Mol. Gen. Genet. 254(3): 337-343; Russell et al. (1997) Transgenic Res. 6(2): 157-168; Rinehart et al (1996) Plant Physiol. 112(3): 1331-1341; Van Camp et al (1996) Plant Physiol. 112(2): 525-535; Canevascni et al (1996) Plant Physiol. 112(2): 513-524; Yamamoto et al (1994) Plant Cell Physiol. 35(5): 773-778; Lam (1994) Results Probl. Cell Differ. 20: 181-196; Orozco et al (1993) Plant Mol. Biol. 23(6): 1129-1138; Mutsuoka et al (1993) Proc. Natl. Acad. Sci. USA 90(20): 9586-9590; and Guevara-Garcia et al (1993) Plant J. 4(3): 495-50.

In a preferred disclosure said tissue specific promoter is a promoter which is active during the accumulation of oil in developing oil seeds, see Broun et al. (1998) Plant J. 13(2): 201-210.

"Operably linked" means joined as part of the same nucleic acid molecule, suitably positioned and oriented for transcription to be initiated from the promoter. DNA operably linked to a promoter is "under transcriptional initiation regulation" of the promoter.

In a preferred embodiment the promoter is an inducible promoter or a developmentally regulated promoter.

Particular vectors are nucleic acid constructs which operate as plant vectors. Specific procedures and vectors previously used with wide success upon plants are described by Guerineau and Mullineaux (1993) (Plant transformation and expression vectors. In: Plant Molecular Biology Labfax (Croy RRD ed) Oxford, BIOS Scientific Publishers, pp 121-148. Suitable vectors may include plant viral-derived vectors (see e.g. EP-A-194809).

Vectors may also include selectable genetic marker such as those that confer selectable phenotypes such as resistance to herbicides (e.g. kanamycin, hygromycin, phosphinotricin, chlorsulfuron, methotrexate, gentamycin, spectinomycin, imidazolinones and glyphosate).

Alternatively, or in addition, said vectors are vectors suitable for mammalian cell transfection or yeast cell transfection. In the latter example multi-copy vectors such as 2µ episomal vectors are preferred. Alternatively yeast CEN vectors and intergrating vectors such as YIP vectors are suitable for transformation of yeast species such as *Saccharomyces cerevisiae* and *Pichia* spp.

It will be apparent to one skilled in the art that a vector may include nucleic acid molecules encoding different enzyme activities to facilitate the delivery of different enzyme activities to a transfected or transformed cell to reconstitute enzymic pathways.

In a preferred embodiment of the invention said cell is transformed with nucleic acid molecules selected from the group consisting of nucleic acid sequences encoding elongase and desaturase and/or acyl-CoA synthetase and/or diacylglycerol acyltransferase activities to provide a cell in which at least part of a 3-n fatty acid biosynthetic pathway is reconstituted.

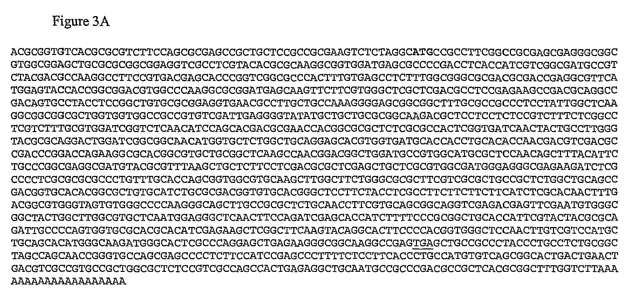

In a further preferred embodiment of the invention said a plant cell of the invention is further transfected with a vector comprising a nucleic acid molecule selected from the group consisting of:
i) a DNA molecules consisting of the DNA sequences as represented in Figures 3a;
ii) DNA molecules which hybridise under stringent hybridization conditions to the sequence identified in (i) above and which have desaturase activity;
iii) DNA molecules comprising DNA sequences that are degenerate as a result of the genetic code to the DNA sequence defined in (i) and (ii) above.

In a preferred embodiment of the invention said cell is selected from the group consisting of: mammalian cells (e.g Chinese Hamster Ovary cells); yeast cells (e.g. *Saccharomyces spp, Pichia* spp); algal cells (e.g *Phaeodactylum tricornutum, Chlamydomonas reinhardtii*); plant cells.

According to a further aspect of the invention there is provided a plant comprising a cell according to the invention.

In a preferred embodiment of the invention said plant is selected from: corn (*Zea mays*), canola (*Brassica napus, Brassica rapa* ssp.), flax (*Linum usitatissimum),* alfalfa (*Medicago sativa*), rice (*Oryza sativa*), rye (*Secale cerale*), sorghum (*Sorghum bicolor, Sorghum vulgare*)*,* sunflower (*Helianthus annus*)*,* wheat (*Tritium aestivum*), soybean (*Glycine max*), tobacco (*Nicotiana tabacum*)*,* potato (*Solanum tuberosum*), peanuts (*Arachis hypogaea*)*,* cotton (*Gossypium hir sutum*), sweet potato (*Iopmoea batatus*), cassava (*Manihot esculenta*), coffee (Cofea spp.), coconut (*Cocos nucifera*), pineapple *(Anana comosus*)*,* citris tree (Citrus spp.) cocoa (*Theobroma cacao*), tea (*Camellia senensis*)*,* banana (*Musa* spp.), avacado (*Persea americana*), fig (*Ficus casica*), guava (*Psidium guajava*)*,* mango (*Mangifer indica*), olive (*Olea europaea*), papaya (*Carica papaya*)*,* cashew (*Anacardium occidentale*)*,* macadamia (*Macadamia intergrifolia*)*,* almond (*Prunus amygdalus*)*,* sugar beets (*Beta vulgaris*), oats, barley, vegetables and ornamentals.

Preferably, plants of the present invention are crop plants (for example, cereals and pulses, maize, wheat, potatoes, tapioca, rice, sorghum, millet, cassava, barley, pea), and other root, tuber or seed crops. Important seed crops are oil-seed rape, sugar beet, maize, sunflower, soybean,sorghum, and flax (linseed). Horticultural plants to which the present invention may be applied may include lettuce, endive, and vegetable brassicas including cabbage, broccoli, and cauliflower. The present invention may be applied in tobacco, cucurbits, carrot, strawberry, sunflower, tomato, pepper.

Grain plants that provide seeds of interest include oil-seed plants and leguminous plants. Seeds of interest include grain seeds, such as corn, wheat, barley, rice, sorghum, rye, etc. Oil-seed plants include cotton, soybean, safflower, sunflower, Brassica, maize, alfalfa, palm, coconut, etc. Leguminous plants include beans and peas. Beans include guar, locust bean, fenugreek, soybean, garden beans, cowpea, mungbean, lima bean, fava been, lentils, chickpea, etc.

It will be apparent that transgenic plants adapted for the production of n-3 fatty acids, in particular DHA, can either be eaten directly or used as a source for the extraction of essential fatty acid, of which DHA would be a constituent.

According to a yet further aspect of the invention there is provided a seed comprising a cell according to the invention.

In a further preferred embodiment of the invention said cell is a yeast cell, preferably of the genus *Saccharomyces spp,* preferably Brewer's yeast *Saccharomyces cerevisiae.*

The genus *Saccharomyces spp* is used in both brewing of beer and wine making and also as an agent in baking, particularly bread. Yeast is a major constituent of vegetable extracts of which Marmite^{tm} is a typical example. Yeast is also used as an additive in animal feed. It will be apparent that genetically engineered yeast strains can be provided which are adapted to synthesise n-3 fatty acids. These yeast strains can then be used in food stuffs and in wine and beer making to provide products which have enhanced n-3 fatty acid content and in particular DHA content.

A foodstuff product comprising a yeast cell according to the invention is disclosed.

In a preferred embodiment said foodstuff product is selected from the group consisting of: wine; beer; bread, baking products (e.g. bread, cake); vegetable extracts.

In a further preferred embodiment said wine or beer in non-alcoholic.

According to a further aspect of the invention there is provided a fermentation process comprising a plant cell according to the invention.

In a preferred embodiment of the invention said fermentation process comprises the steps of :
i) providing a vessel containing a cell according to the invention and constituents required for fermentation and fatty acid biosynthesis; and
ii) providing conditions conducive to the fermentation of the liquid composition contained in said vessel.

A method of modulating the level of n-3 fatty acid, in particular DHA, or variants thereof, in a plant cell comprising;
i) providing a plant cell according to the invention;
ii) regenerating the plant cell into a plant; and
iii) monitoring n-3 fatty acid production by said plant is disclosed.

A method for the production and optionally the extraction of n-3 fatty acids, in particular DHA, comprising:
i) providing a cell according to the invention;
ii) providing conditions conducive to the growth of said cell; and
iii) extracting n-3 fatty acids, or variants thereof, from said cell is disclosed.

According to a yet further aspect of the invention there is provided a method for the production and optionally the extraction of n-3 fatty acid, particularly DHA, comprising:
i) providing a plant cell according to the invention;
ii) regenerating said cell into a plant; and
iii) extracting n-3 fatty acids from said plant.

N-3 fatty acids, particularly DHA, or variants thereof, obtainable by the method(s) according to the invention is disclosed.

N-3 fatty acid, or variant thereof, for use in the manufacture of a medicament for use in the treatment of conditions which would benefit from administration of n-3 fatty acids, or variant thereof is disclosed.

In a preferred embodiment of the invention said condition is selected from the group consisting of: cardiac arrhythmia's; rheumatoid arthritis; Crohn's disease; schizophrenia; cancer; foetal alcohol syndrome; attention deficient hyperactivity disorder; cystic fibrosis; phenylketonuria; unipolar depression; aggressive hostility; adrenoleukodystophy; coronary heart disease, hypertension, type II diabetes, ocular diseases.

A non-human transgenic animal comprising at least one nucleic acid molecule according to the invention is disclosed.

A reaction vessel comprising at least one polypeptide disclosed herein fatty acid substrates and co-factors characterised in that said vessel is adapted for the conversion of said fatty acids substrates to n-3 fatty acids, in particular docosahexaenoic acid is disclosed.

In a preferred embodiment said vessel comprises polypeptides having elongase, desaturase, acyl-CoA synthetase and diacylglycerol acyltransferase activities to provide a vessel in which at least part of a 3-n fatty acid biosynthetic pathway is reconstituted.

In a further preferred embodiment said polypeptides are those protein molecules disclosed herein. In particular, protein molecules which comprise the sequences as represented by Figures 2a, 2b, 2c, 3b, 4b, 5b or 6b.

In a preferred embodiment said at least one polypeptide is expressed by a cell according to the invention.

In a preferred embodiment said polypeptide(s) is/are soluble. Alternatively said polypeptide(s) is/are immobilised.

In a further preferred embodiment said vessel is a bioreactor.

It will be apparent to one skilled in the art that a polypeptide disclosed herein has utility with respect to the *in vivo* biosynthesis of n-3 fatty acids through transformation or transfection of nucleic acids encoding said polypeptide(s) into suitable host cells. Fatty acids can then either be extracted from said cells or foods comprising said cells can be eaten. Cells expressing said polypeptide (s) can also be incubated under suitable growth conditions to facilitate the synthesis of fatty acids. Alternatively, said polypeptide (s) can either be purified from an algal cell culture or manufactured recombinantly and used in a bioreactor to synthesise fatty *acids in vitro.*

An embodiment of the invention will now be described by example only and with reference to the following figures:
Figure 1a represents the nucleic acid sequence of a nucleic acid molecule comprising a fatty acid elongase TpELO2.1; Reference Figure 1b the nucleic acid sequence of the fatty acid elongase TpELO2.2; Reference Figure 1c the nucleic acid sequence of the fatty acid elongase TpELO2.3;.
Figure 2a represents the amino acid sequence of TpELO2.1; Reference Figure 2b represents the amino acid sequence of TpELO2.2; and Reference Figure 2c represents the amino acid sequence of TpELO2.3;
Figure 3a represents the nucleic acid sequence of *PIDES1;* Figure 3b represents the amino acid sequence of *PIDES1;.*
Reference Figure 4a represents the nucleic acid sequence of a nucleic acid molecule comprising fatty acid desaturase, *PIDES2;* Reference Figure 4b the amino acid sequence comprising *PIDES2;*
Reference Figure 5a represents the nucleic acid sequence of a nucleic acid molecule comprising acyl-CoA synthetase, *PIACS1;* Reference Figure 5b the amino acid sequence comprising *PIACS1;*
Reference Figure 6a the full length sequence of a nucleic acid molecule encoding *PIDGAT2-1;* Reference Figure 6b the full length amino acid sequence of *PIDGAT2-1* polypeptide; and
Reference Figure 7a is the nucleic acid sequence of PIELO1; Reference Figure 7b amino acid sequence of PIELO 1 ; Reference Figure 7c is the nucleic acid sequence of PIELO 2; Reference Figure 7d is the amino acid sequence of PIELO 2.

### MATERIALS AND METHODS

### Cultivation of Pavlova lutheri

*Pavlova lutheri* (CCAP 931/1) was obtained from the Culture Collection of Algae and Protozoa (Dunstaffnage Marine Lab., Oban, PA34 4AD, Scotland, U.K.).

The growth medium used in all experiments was enriched artificial seawater medium (EASW), made up in 20 1 batches as described by Harrison et *al.* (1980), and modified by Thompson et *al.* (1991). The medium was further modified by increasing the macronutrient concentrations of NaNO₃ and Na₂SiO₃.9H₂O to 1 mM, and NaH₂PO₄ to 200 µM. The silicate was dissolved separately in deionized distilled water and the pH adjusted to approximately 8.0 with 50% HCl before it was added to the medium. This medium was buffered to pH 8.0 by adding 20 mM N-[2-hydroxyethyl]piperazine-N'-[2-ethanesulfonic acid] (HEPES) and 20 mM NaOH. The freshly prepared medium was filtered through a 0.22 µM Millipore™ GS membrane filter into a 20 1 sterile propylene reservoir. It was then dispatched by 0.5 1 in 1 1 conical glass flasks and sterilized by autoclaving (30 min, 120°C). The batch cultures were grown at 15°C with 50 µE m⁻² s⁻¹ constant illumination, and aeration provided by shaking the flasks at 150 rpm.

Cell density was monitored by counting cells with a haemacytometer. Since the *Pavlova lutheri* cells are motile, they were first incubated in sodium azide 20 mM to immobilise before counting.

The nitrate concentration was determined periodically during the culture time by measuring the change of the medium absorbance at 220 nm, according to the method described by Collos et *al.* (1999).

### Isolation of total and poly(A)⁺ RNA from P. lutheri

The algal culture was harvested by centrifugation at 4,500 rpm for 15 min. The cell pellet was suspended in 1 volume of extraction buffer (25 mM Tris-HCl pH 8.0, 25 mM EDTA pH 8.0, 75 mM NaCl, 1 % SDS v/v, 7.8 % β-mercaptoethanol v/v, in DEPC treated water), and one volume of 25:24:1 phenol:chloroform:isoamyl alcohol (v/v) was added. After 13,000 rpm centrifugation at 4 °C for 10 min, the aqueous phase was transferred to a new tube and 1 volume of 24:1 chloroform:isoamyl alcohol v/v added. After a second round of centrifugation, the upper phase was transferred to a fresh tube and LiCl added to a final concentration of 2 M. This solution was incubated for 1hr at -20 °C, and then centrifuged at 13,000 rpm at 4 °C for 15 min. The resulting pellet was re-suspended in DEPC treated water and the RNA was precipitated by addition of 0.1 volume of 3 M sodium acetate, pH 5.5 and 2.5 volumes of absolute ethanol followed by incubation for 20 min at 4 °C. This sample was then centrifuged at 13,000 rpm at 4 °C for 15 min and the resulting pellet was washed with 70 % ethanol, dried and re-suspended in DEPC treated water. Quantity and quality of the extract were estimated by measuring optical density at 260 and 280 nm (1 O.D._{260 nm} = 40 µg/ml RNA). An aliquot of the extract was also visualised on a 1 % (w/v) agarose gel containing ethidium bromide.

For the cDNA library construction, poly(A)⁺ RNA was prepared with the Poly(A) Quick® mRNA isolation kit (Stratagene) from cells harvested during the exponential phase.

### cDNA library construction and pBluescript phagemid excision

Double-stranded, end-adapted cDNA synthesised using a cDNA synthesis kit (Stratagene) was passed through a Sepharose CL-2B gel filtration column (Stratagene) to remove adapters and small cDNA molecules. cDNA eluted from the column was phenol-extracted, ethanol-precipitated and ligated to arms of the Uni-ZAP XR Vector (Stratagene) before packaging into λ phage using the Gigapack III Gold Packaging Extract (Stratagene). A primary library of 3 X 10⁶ plaque forming units (pfu) was obtained with the majority of the inserts examined ranging from 0.3 to 1.5 kb. The library was subsequently amplified.

After amplification, the cDNA library was excised using the ExAssist™ Interference-Resistant Helper Phage (Stratagene). The excised phagemids were plated as individual bacterial colonies following the manufacturer's instructions. The presence of insert was checked by PCR using universal primers and clones containing cDNA longer than 0.2 kb were selected for sequencing.

### Sequencing and sequence analysis

Sequencing reactions were prepared with the ABI Prism Big Dye-Terminator cycle sequencing kit (PE Applied Biosystems), using the universal T3 primer, and these were run on an ABI3700 (96-capillaries) sequencer (PE Applied Biosystems). The resulting Expressed Sequence Tags (ESTs) were processed such that all vector sequences were removed and further examined using standard nucleotide sequence alignment algorithms in order to identify EST clones with overlapping sequences. These overlapping sequences were then assembled into contigs. These contigs were annotated by comparison with the non-redundant peptide and nucleotide databases available from the National Centre for Biotechnology Information (NCBI). The NCBI databases had been downloaded to a local Silicon Graphics Server which allowed the annotation of thousands of ESTs to be performed as a batch job using the BLAST 2 sequence alignment algorithm (Altschul *et al.,* 1997).

The *PIELO1 PlELO2, PIDES1*, *PIDES2, PIACS1* and *PIDGAT2-1* clones were identified on the basis of homology with other fatty acid elongase/desaturase/acyl-CoA synthase or diacylglycerol acyltransferase genes in the NCBI nucleotide and protein databases.

### Hybridisation conditions

Stringent hybridisation/washing conditions are well known in the art. For example, nucleic acid hybrids that are stable after washing in 0.1xSSC,0.1% SDS at 60°C. It is well known in the art that optimal hybridisation conditions can be calculated if the sequence of the nucleic acid is known. Typically, hybridisation conditions uses 4 - 6 x SSPE (20x SSPE contains 175.3g NaCl, 88.2g NaH₂PO₄ H₂O and 7.4g EDTA dissolved to 1 litre and the pH adjusted to 7.4); 5-10x Denhardts solution (50x Denhardts solution contains 5g Ficoll (Type 400, Pharmacia), 5g polyvinylpyrrolidone and 5g bovine serum albumen); 100µg-1.0mg/ml sonicated salmon/herring DNA; 0.1-1.0% sodium dodecyl sulphate; optionally 40-60% deionised formamide. Hybridisation temperature will vary depending on the GC content of the nucleic acid target sequence but will typically be between 42°- 65° C.

### Functional analysis of by heterologous expression.

Functional characterisation of the amino acid sequence encoded by *TpELO1.2, 2.2* and 2.3 will be performed under protocols previously described (Jaworski *et al.,* 2001(or refer to as: US Patent No. 6,307,128);Qi *et al.,* 2002). To this aim, several species of fatty acid substrates will be considered: saturated (16:0, 18:0, 20:0, 22:0), monounsaturated (16:1, 18:1, 20:1) and polyunsaturated (20:4n-6, 20:5n-3, 22:5n-3.

Functional characterisation of the amino acid sequence encoded by *PlDES1* and *PIDES2* will be performed under protocols previously described (Qiu *et al.,* 2001). To this aim, several species of fatty acid substrates will be considered: saturated (16:0, 18:0), monounsaturated (16:1, 18:1) and polyunsaturated (18:2n-6, 18:3n-3, 18:3n-6, 18:4n-3; 20:2n-6, 20-3n-3; 20:3n-6, 20:4n-3, 20:4n-6, 20:5n-3, 22:4n-6, 22:5n-3 and 22:5n-6).

Functional characterisation of the amino acid sequence encoded by *PlACS1* will be performed under protocols previously described (Kang *et al.,* 1997). To this aim, several species of fatty acid substrates will be considered: saturated (8:0, 10:0, 12:0, 16:0, 18:0, 20:0, 22:0), monounsaturated (14:1, 16:1, 18:1) and polyunsaturated (18:2n-6, 18:3n-3, 18:3n-6, 20:4n-6, 20:5n-3 and 22:6n-3).

Functional characterisation of the amino acid sequence encoded by *PIDGAT2-1* will be performed under protocols previously described (Lardizabal *et al.,* 2001; Cases *et al.,* 2001, Zou *et al.,* 1999). To this aim, DGAT activity will be assayed by incorporation of [1-¹⁴ C] diacylglycerol into TAG in the presence of several species of fatty acyl CoA substrates that are representative of fatty acids that partition to TAG in *P. lutheri.* These include: 14:0, 16:0, 16:1, 18:0, 18:1, 18:2, 18:4, 20:5 and 22:6.

### TAG extraction and fatty acids analysis

The alga cells (2 ml of culture medium) were harvested during the experimental period by centrifugation at 13,000 rpm for 15 min. Fifty µg of tripentadecanoin (15:0-TAG) were added to the pellet as an internal standard. The pellet was then suspended in 1 ml of 2:1 chloroform:methanol (v/v) and frozen in liquid nitrogen. After 1 hour at 4°C, the cell debris was discarded by centrifugation and 0.3 ml of 0.9% KCl added to the supernatant. After centrifugation, the bottom phase was transferred into a 2 ml Ependorf and the KCl rinsed with 0.5 ml of chloroform. The chloroform phases were pooled and dried. The FA extract was suspended in 0.2 ml of hexane, and this volume was divided in 2 fractions of 0.1 ml. The first fraction was dried, and the lipid extract suspended in 0.2 ml of hexane. This represented the total lipid extract. The second fraction was used to isolate the TAGs by hydrophobic chromatography. Bond Elut (Varian) 1 ml solid phase extraction columns with 100 mg Si packing were used to partition TAGs from other lipids in algal extracts. This protocol was adapted from a method described by Yongmanitchai and Ward (1992). The eluate was dried and the TAG extract suspended in 0.2 ml of hexane. The products of these two extractions were analysed by GC as described previously by Larson and Graham (2001).

The same methodology will be employed to extract lipids and fatty acids from yeast cells in order to perform the functional analysis of TpELO 2.1, 2.2 and 2.3 and *PIDES 1* following the feeding of different fatty acids as outlined above.

### Cloning and characterization of the genes PlELO1 , PlELO2, PlDES 1, PlDES 2, PlACS1. PlDGAT2-1, TpELO2.1, 2.2 and 2.3

The first pass sequencing of 5,719 cDNA clones from a cDNA library prepared from *P. lutheri* resulted in the identification of 34 cDNA clones from a single gene which gives a predicted amino acid sequence that has significant identity with fatty acid elongase genes from a variety of organisms. This abundance of copies of the elongase gene indicates that it is expressed at a significant level in *P. lutheri* cells that are producing DHA.

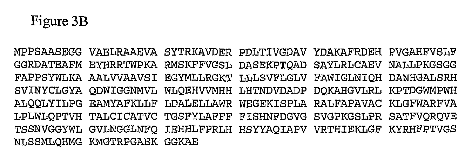

The sequencing of 5,719 cDNA clones from the *P. lutheri* library also resulted in the identification of four cDNA clones from a single gene which gives a predicted amino acid sequence that has significant identity with fatty acid desaturase genes from a variety of organisms (Figure 3a and 3b). This desaturase gene has been designated *PIDES 1.*

The sequencing of 5,719 cDNA clones from the *P. lutheri* library also resulted in the identification of three cDNA clones from a single gene which gives a predicted amino acid sequence that has significant identity with fatty acid desaturase genes from a variety of organisms (Reference Figures 4a and 4b). This desaturase gene has been designated *PlDES 2.*

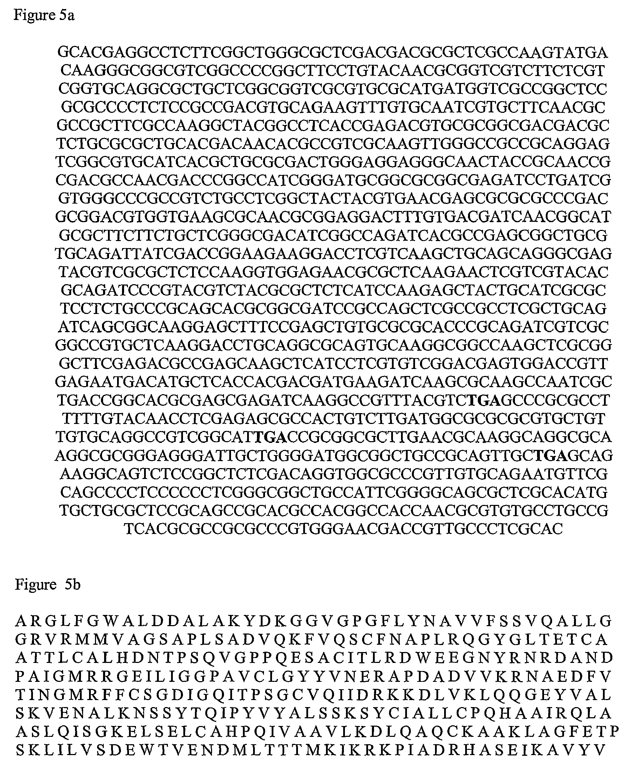

The sequencing of 5,719 cDNA clones from the *P. lutheri* library also resulted in the identification of twelve cDNA clones from a single gene which gives a predicted amino acid sequence that has significant identity with acyl-CoA synthetase genes from a variety of organisms (Reference Figure 5a and 5b). This acyl-CoA synthetase gene has been designated *PlACS1.*

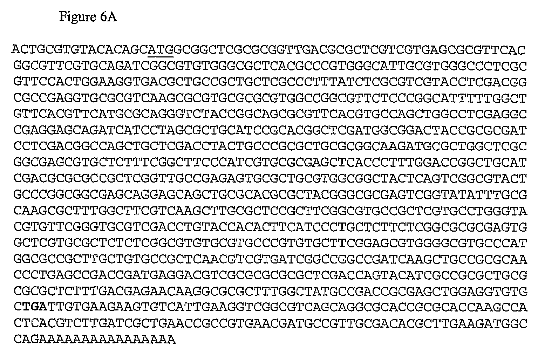

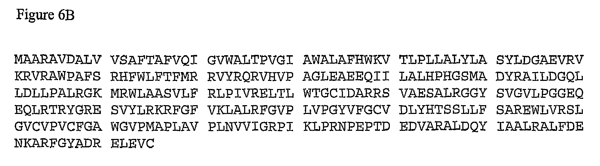

The sequencing of 5,719 cDNA clones from the *P. lutheri* library also resulted in the identification of one cDNA clone which gives a predicted amino acid sequence that has significant identity with diacylglycerol acyltransferase 2 genes from several organisms (Reference Figure 6a and 6b). This diacylglycerol acyltransferase 2 gene has been designated *PlDGAT2-1.*

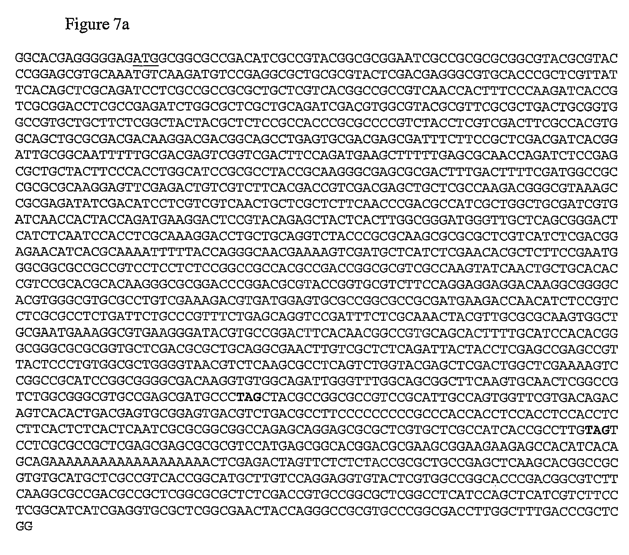

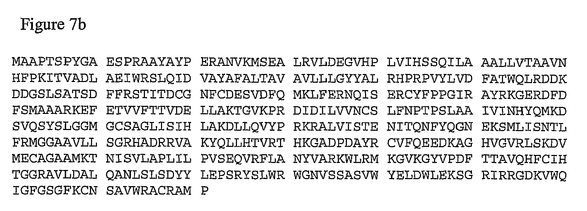

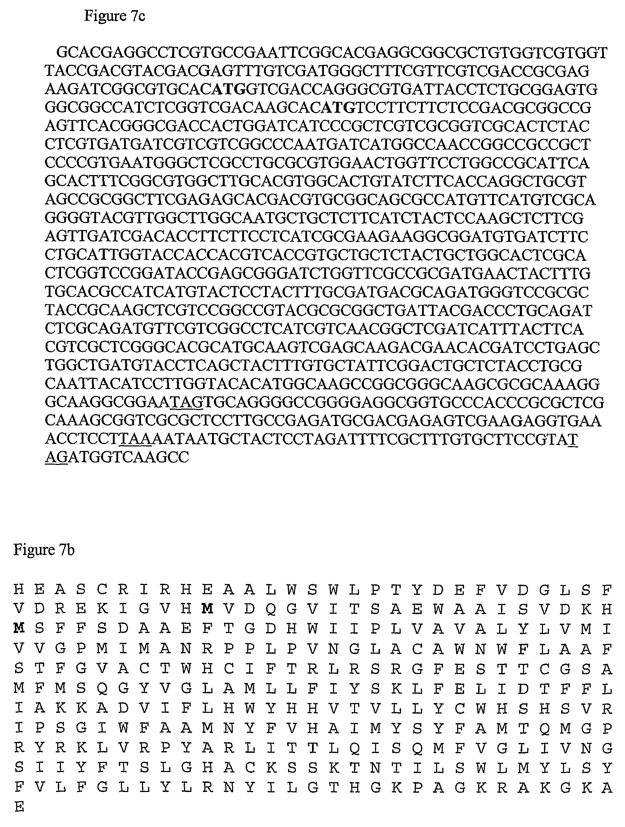

The full length cDNA and protein sequence of *PlELO1 and PlELO2* is disclosed in Figures 7a, 7b, 7c and 7d respectively.

The Tp ELO2.1, 2.2 and 2.3 genes were identified by screening a *Thalassiosira spp* genomic database which can be found at http://www.jai.doe.gov/ with PlELO 1 and PIELO 2.

### REFERENCES

1. Uauy R, Mena P, Rojas C Essential fatty acids in early life: structural and functional role. Proc Nutr Soc 2000 Feb;59(1):3-15
2. Dewailly E, Blanchet C, Lemieux S, Sauve L, Gingras S, Ayotte P, Holub BJ. n-3 Fatty acids and cardiovascular disease risk factors among the Inuit of Nunavik. Am J Clin Nutr 2001 Oct;74(4):464-73
3. Salem N et al. (1994) "Arachidonate and docosahexaenoate biosynthesis in various species and compartments in vivo." World Review of Nutrition and Dietetics, vol. 75, pp. 114-119.
4. Haag, M. Depressive Symptoms in Schizophrenia. The Medicine Journal, November 2001. Page 1-7.
5. Qi B, Beaudoin F, Fraser T, Stobart AK, Napier JA, Lazarus CM. Identification of a cDNA encoding a novel C18-Delta(9) polyunsaturated fatty acid-specific elongating activity from the docosahexaenoic acid (DHA)-producing microalga, Isochrysis galbana. FEBS Lett 2002 Jan 16;510(3): 159-65
6. Ashford A, Barclay WR, Weaver CA, Giddings TH, Zeller S. Electron microscopy may reveal structure of docosahexaenoic acid-rich oil within Schizochytrium sp. Lipids 2000 Dec;35(12):1377-86
7. Hammond BG, Mayhew DA, Robinson K, Mast RW, Sander WJ. Safety assessment of DHA-rich microalgae from Schizochytrium sp. Regul Toxicol Pharmacol 2001 Jun;33(3):356-62
8. Simonpolous AP. Essential fatty acids in health and chronic disease. Am. J. Clin. Nutr. 1999,70 (3); 560S-569S.
9. Horrocks LA & Yeo YK. Health benefits of docosahexaenoic acid (DHA). Pharmacol. Res. 1999;40(3):211.
10. Collos, Y., Mornet, F., Sciandra, A., Waser, N., Larson, A., Harrison, P.J., 1999. An optical method for the rapid measurement of micromolar levels of nitrate in marine phytoplankton cultures. Journal of Applied Phycology 11, 179-184.
11. Harrison, P.J., Waters, R.E., Taylor, F.J.R., 1980. A broad spectrum artificial seawater medium for coastal and open ocean phytoplankton. Journal of Phycology 16,28-35.
12. Larson, T.R. , Graham LA., 2001. A novel technique for the sensitive quantification of acylCoA esters from plant tissues. The Plant Journal 25, 155-125.
13. Thompson, P.A., Harrison, P.J. , Parslow, J.S., 1991. Influence of irradiance on cell volume and carbon quota for ten species of marine phytoplankton. Journal of Phycology 27, 351-360.
14. Yongmanitchai, W., Ward, O.P., 1992. Separation of lipid classes from Phaeodactylum tricornutum using silica cartridges. Phytochemistry 31, 3405-340.

Broun P, Boddupalli S, Somerville C. 1998. A bifunctional oleate 12-hydroxylase: desaturase from Lesquerella fendleri. Plant J 13(2): 201-10
Schnurr JA, Shockey J, Browse J. 2000 Characterization of an acyl-CoA synthetase from Arabidopsis thaliana. Biochem Soc Trans. Dec;28(6):957-8.
Kang MJ, Fujino T, Sasano H, Minekura H, Yabuki N, Nagura H, Iijima H, Yamamoto TT. 1997 A novel arachidonate-preferring acyl-CoA synthetase is present in steroidogenic cells of the rat adrenal, ovary, and testis. Proc Natl Acad Sci USA. Apr 1;94(7):2880-4.
Qiu X, Hong H, MacKenzie SL. 2001 Identification of a Delta 4 fatty acid desaturase from Thraustochytrium sp. involved in the biosynthesis of docosahexanoic acid by heterologous expression in Saccharomyces cerevisiae and Brassica juncea. J Biol Chem. Aug 24;276(34):31561-6.
Cases S, Smith SJ, Zheng YW, Myers HM, Lear SR, Sande E, Novak S, Collins C, Welch CB, Lusis AJ, Erickson SK, Farese RV Jr. 1998. Identification of a gene encoding an acyl CoA:diacylglycerol acyltransferase, a key enzyme in triacylglycerol synthesis. Proc Natl Acad Sci USA. Oct 27;95(22):13018-23.
Hobbs DH, Lu C, Hills MJ. 1999. Cloning of a cDNA encoding diacylglycerol acyltransferase from Arabidopsis thaliana and its functional expression. FEBS Lett. 11;452(3):145-9.
Jako C, Kumar A, Wei Y, Zou J, Barton DL, Giblin EM, Covello PS, Taylor DC. 2001. Seed-specific over-expression of an Arabidopsis cDNA encoding a diacylglycerol acyltransferase enhances seed oil content and seed weight. Plant Physiol. 126(2):861-74.
Lardizabal KD, Mai JT, Wagner NW, Wyrick A, Voelker T, Hawkins DJ. 2001. DGAT2 is a new diacylglycerol acyltransferase gene family: purification, cloning, and expression in insect cells of two polypeptides from Mortierella ramanniana with diacylglycerol acyltransferase activity. J Biol Chem. 19;276(42):38862-9.
Cases S, Stone SJ, Zhou P, Yen E, Tow B, Lardizabal KD, Voelker T, Farese RV Jr. 2001. Cloning of DGAT2, a second mammalian diacylglycerol acyltransferase, and related family members. J Biol Chem. Oct 19;276(42):38870-6.
Zou J, Wei Y, Jako C, Kumar A, Selvaraj G, Taylor DC. 1999. The Arabidopsis thaliana TAG1 mutant has a mutation in a diacylglycerol acyltransferase gene. Plant J. 19(6):645-53.

## Claims

1. A transgenic plant cell transfected with a vector comprising a nucleic acid molecule selected from the group consisting of:
i) a DNA moleculeselected from the DNA sequences as represented in Figure 1 a; and
ii) a DNA molecule which anneals under stringent hybridization conditions to the sequences identified in (i) above and which encode a polypeptide which has fatty acid elongase activity.

2. A plant cell according to claim 1 wherein said polypeptide is a variant polypeptide having fatty acid elongase activity and comprises the amino acid sequence represented in Figure 2a which sequence has been modified by deletion, addition or substitution of at least one amino acid residue and has at least 75% sequence identity with the amino acid sequence in Figure 2a wherein said modification enhances the enzyme activity of said polypeptide.

3. A plant cell according to claim 1 or 2 wherein said polypeptide comprises the amino acid sequence represented in Figures 2a.

4. A cell according to claim 7 wherein said polypeptide consists of the amino acid sequence represented in Figures 2a.

5. A plant cell according to any of claims 1-4 wherein said cell is further transfected with a vector comprising a nucleic acid molecule selected from the group consisting of: :
i) a DNA molecule consisting of the DNA sequence as represented in Figure 3a;
ii) DNA molecules which hybridise under stringent hybridization conditions to the sequence identified in (i) above and which have desaturase activity;
iii) DNA molecules comprising DNA sequences that are degenerate as a result of the genetic code to the DNA sequence defined in (i) and (ii) above.

6. A plant comprising a cell according to any of claim 1-5

7. A seed comprising a cell according to any of claims 1-5.

8. A fermentation process comprising a plant cell according to any of claims 1-5.

9. A fermentation process according to claim 8 said process comprises the steps of:
i) providing a vessel containing a cell according to any of claims 1-5 and constituents required for fermentation and fatty acid biosynthesis; and
i) providing conditions conducive to the fermentation of the liquid composition contained in said vessel.

10. A method for the production and optionally the extraction of n-3 fatty acid comprising:
i) providing a plant cell according to any of claims 1-5;
ii) regenerating said cell into a plant; and
i) extracting n-3 fatty acids from said plant.

11. Use of the plant cell of any one of claims 1 to 5 for the production of a foodstuff product or a feedstuff product.

## Patentansprüche

1. Transgene Pflanzenzelle, die mit einem Vektor transfiziert ist, der ein Nukleinsäuremolekül umfasst, das ausgewählt ist aus der Gruppe, bestehend aus:
i) einem DNA-Molekül, ausgewählt aus den in Figur 1a dargestellten DNA-Sequenzen; und
ii) einem DNA-Molekül, das unter stringenten Hybridisierungsbedingungen an die in (i) oben identifizierten Sequenzen hybridisiert, und das ein Polypeptid codiert, das eine Fettsäureelongase-Aktivität aufweist.

2. Pflanzenzelle nach Anspruch 1, wobei das Polypeptid ein variantes Polypeptid mit Fettsäureelongase-Aktivität ist, und das die in Figur 2a veranschaulichte Aminosäuresequenz umfasst, wobei die Sequenz durch Deletion, Addition oder Substitution von mindestens einem Aminosäurerest modifiziert worden ist und mindestens 75% Sequenzidentität zur Aminosäuresequenz in Figur 2a hat, wobei die Modifikation die Enzymaktivität des Polypeptids steigert.

3. Pflanzenzelle nach Anspruch 1 oder 2, wobei das Polypeptid die in den Figuren 2a veranschaulichte Aminosäuresequenz umfasst.

4. Zelle nach Anspruch 7, wobei das Polypeptid aus der in den Figuren 2a veranschaulichten Aminosäuresequenz besteht.

5. Pflanzenzelle nach einem der Ansprüche 1 bis 4, wobei die Zelle zudem mit einem Vektor transfiziert ist, umfassend ein Nukleinsäuremolekül, das ausgewählt ist aus der Gruppe, bestehend aus:
i) einem DNA-Molekül, bestehend aus der in Figur 3a veranschaulichten DNA-Sequenz;
ii) DNA-Molekülen, die unter stringenten Hybridisierungsbedingungen an die in (i) oben identifizierte Sequenz hybridisieren und die Desaturase-Aktivität aufweisen;
iii) DNA-Molekülen, umfassend DNA-Sequenzen, die aufgrund des genetischen Codes an der in (i) und (ii) oben definierten DNA-Sequenz degeneriert sind.

6. Pflanze, umfassend eine Zelle nach einem der Ansprüche 1 bis 5.

7. Samen, umfassend eine Zelle nach einem der Ansprüche 1 bis 5.

8. Fermentationsprozess, umfassend eine Pflanzenzelle nach einem der Ansprüche 1 bis 5.

9. Fermentationsverfahren nach Anspruch 8, wobei das Verfahren die folgenden Schritte umfasst:
i) Bereitstellen eines Behälters, der eine Zelle nach einem der Ansprüche 1 bis 5, und Bestandteile, die zur Fermentation und Fettsäurebiosynthese erforderlich sind, enthält; und
i) Bereitstellen von Bedingungen, die für die Fermentation der in dem Behälter befindlichen flüssigen Zusammensetzung förderlich sind.

10. Verfahren zur Herstellung und gegebenenfalls der Extraktion von n-3-Fettsäure, umfassend:
i) Bereitstellen einer Pflanzenzelle nach einem der Ansprüche 1 bis 5;
ii) Regenerieren der Zelle in eine Pflanze; und
i) Extrahieren der n-3-Fettsäuren aus der Pflanze.

11. Verwendung der Pflanzenzelle nach einem der Ansprüche 1 bis 5 zur Produktion eines Nahrungsmittelprodukts oder eines Futtermittelprodukts.

## Revendications

1. Cellule végétale transgénique transfectée par un vecteur comprenant une molécule d'acide nucléique choisie dans le groupe constitué par
i) une molécule d'ADN choisie parmi les séquences d'ADN telles que représentées à la Figure 1a ; et
ii) une molécule d'ADN qui s'annelle dans des conditions d'hybridation stringentes avec les séquences identifiées dans (i) ci-dessus et qui codent pour un polypeptide ayant une activité d'élongase d'acides gras.

2. Cellule végétale selon la revendication 1, **caractérisée en ce que** ledit polypeptide est un polypeptide variant ayant une activité d'élongase d'acides gras et comprend la séquence d'acides aminés représentée à la Figure 2a, laquelle séquence a été modifiée par délétion, addition ou substitution d'au moins un résidu d'acide aminé et possède une identité de séquence d'au moins 75% avec la séquence d'acides aminés de la Figure 2a, où ladite modification améliore l'activité enzymatique dudit polypeptide.

3. Cellule végétale selon la revendication 1 ou 2, **caractérisée en ce que** ledit polypeptide comprend la séquence d'acides aminés représentée aux Figures 2a.

4. Cellule végétale selon la revendication 7, **caractérisée en ce que** ledit polypeptide est constitué de la séquence d'acides aminés représentée aux Figures 2a.

5. Cellule végétale selon l'une quelconque des revendications 1-4, **caractérisée en ce que** ladite cellule est en outre transfectée par un vecteur comprenant une molécule d'acide nucléique choisie dans le groupe constitué par
i) une molécule d'ADN constituée de la séquence d'ADN telle que représentée à la Figure 3a ;
ii) des molécules d'ADN qui s'hybrident dans des conditions d'hybridation stringentes avec la séquence identifiée dans (i) ci-dessus et qui possèdent une activité de désaturase ;
iii) des molécules d'ADN comprenant des séquences d'ADN qui sont dégénérées à cause du code génétique en séquence d'ADN définie dans (i) et (ii) ci-dessus.

6. Plante comprenant une cellule selon l'une quelconque des revendications 1-5.

7. Graine comprenant une cellule selon l'une quelconque des revendications 1-5.

8. Procédé de fermentation comprenant une cellule végétale selon l'une quelconque des revendications 1-5.

9. Procédé de fermentation selon la revendication 8, ledit procédé comprenant les étapes consistant à
i) fournir un récipient contenant une cellule selon l'une quelconque des revendications 1-5 et des constituants nécessaires pour la fermentation et la biosynthèse d'acides gras ; et
i) fournir des conditions favorables à la fermentation de la composition liquide contenue dans ledit récipient.

10. Méthode de production et éventuellement d'extraction d'acides gras n-3, comprenant
i) la fourniture d'une cellule végétale selon l'une quelconque des revendications 1-5 ;
ii) la régénération de ladite cellule en une plante ; et
i) l'extraction d'acides gras n-3 à partir de ladite plante.

11. Utilisation de la cellule végétale selon l'une quelconque des revendications 1 à 5, pour la production d'un produit d'alimentation humaine ou d'un produit d'alimentation animale.
